Europäisches Patentamt

European Patent Office

Office européen des brevets

(11) Publication number: **0 322 146**
**A2**

## EUROPEAN PATENT APPLICATION

(21) Application number: 88311790.5

(22) Date of filing: 13.12.88

(51) Int. Cl.4: **A61K 39/00** , **C12P 21/00** ,
**C12N 15/00** , **G01N 33/574** ,
**G01N 33/577**

The applicant has filed a statement in accordance with Rule 28 (4) EPC (issue of a sample only to an expert). Accession number(s) of the deposit(s): ECACC 87121101

(30) Priority: 16.12.87 GB 8729304
08.07.88 GB 8816272

(43) Date of publication of application:
28.06.89 Bulletin 89/26

(84) Designated Contracting States:
BE CH DE ES FR GB GR IT LI NL SE

(71) Applicant: LYNXVALE LIMITED
The Old Schools
Cambridge CB2 ITS(GB)

(72) Inventor: Metcalfe, Susan Marie
10 Fendon Road
Cambridge(GB)
Inventor: Davies, Hugh Ffrangcon Stuart
106 Cambridge Road
Great Shelford Cambridge CB2 5JS(GB)

(74) Representative: Perry, Robert Edward et al
GILL JENNINGS & EVERY 53-64 Chancery Lane
London WC2A 1HN(GB)

(54) **Antibody and antigen.**

(57) An antibody which is useful in the diagnosis of cancer and a complementary antigenic compound (as obtainable from humans where, in vivo, it is present as a minor component on the surface of erythrocytes, and which becomes enhanced in cancer patients), having the following characteristics:
soluble in 1% deoxycholate;
monoepitopic for wheat germ agglutinin (Triticum vulgaris);
binds Phytolacca americana and Triticum vulgaris;
does not bind Arachis hypogea, Helix pomatia, Mycoplasma gallisepticum or Tetragonolobus paupureas;
trypsin-sensitive;
molecular weight (monomer) of approx. 35,000 on sodium dodecylsulphate-polyacrylamide gel electrophoresis (SDS-PAGE);
silver stain on analytical acrylamide gel; and
protein only just detectable (Petersen modification of Lowry method).

EP 0 322 146 A2

## ANTIBODY AND ANTIGEN

### FIELD OF THE INVENTION

This invention relates to an antigen which is a tumour marker, to antibodies to the antigen, and to the diagnostic use of the antibodies.

### PRIOR ART

Tumour markers are known. A particular marker is described by Metcalfe et al, Br. J. Cancer (1984) 49, Annal. Royal Coll. Surgeons England (1984) 66 399-401, Br. J. Cancer (July 1985) 52(1) 127-129, and Urol. Int. (1987) 42 254-259. This marker is valuable because it is enhanced in cancer patients, apparently regardless of tumour type, and this can be detected by the molecular property of an antibody (which has been named B5) to cause haemagglutination by binding an antigenic epitope on the surface of human erythrocytes. The B5 antibody is described in the given publications, e.g. as a rat monoclonal antibody (IgG), and by Milner & Metcalfe, Lancet (1982) 2 1100-1.

The Br. J. Cancer (1984) article, supra, describes the B5 antibody as a product of fusion between Y3-Agl.2.3 rat myeloma cells (Galfre et al, Nature 277 131; see also EP-A-0014519) and spleen cells from an AO rat immunised with desialylated human erythrocytes (blood group O). Each immunising dose was of 10⁹ cells, the priming dose being in complete Freund's adjuvant and intramuscular (i.m.). The first i.m. boost was in incomplete Freund's and the second i.m. boost was without adjuvant, each being at 4 week intervals. Three days after a final i.v. boost, spleen cells were collected and fused with the Y3 cells using polyethylene glycol (B.D.H., m.w. 1500) by standard procedure. Those supernatants which caused agglutination of desialylated, but not whole, erythrocytes were regarded as positive. In the fusion from which B5 was obtained, B5 was the only positive clone; in subsequent fusions, a large number of positive supernatants were found, but none had the binding properties of B5.

In the same article, the "B5 antigen" is described in part, e.g. as a surface antigen sensitive to trypsin and tightly associated with the membrane. In addition to giving various results, the article concludes "that B5 detects a previously unknown, indirect marker of malignant tumours. We have no reason to suspect that B5-haemagglutination is caused by therapy since the incidence of B5 positive samples was the same in untreated patients, as in those already on chemotherapy and/or radiotherapy. In serial studies our finding that some tumour patients who had been consistently B5 positive became B5 negative towards the end of therapy, encourages us to believe that such a switch from positive to negative may indicate successful treatment of tumour. Since the only requirement is a small blood sample, B5 provides a very simple, non-invasive test applicable to a wide range of malignancies. We feel its greatest worth will be in the monitoring of individuals for tumour status, both for tumour regression during therapy, and for tumour recurrence during follow-up."

### SUMMARY OF THE INVENTION

While the prior art does not give a reproducible method for the production of either B5 antigen or antibody, respective materials of the same type are characterised herein. The antibody particular is of a unusual class, i.e. a rat immunoglobin in monoclonal form, class IgG subclass 2c, with λ light chains. The antigen (which has been isolated) is of potential value in revealing enzymic characteristics of tumour which may be exploited in the detection and/or treatment of cancer.

A more general aspect of the invention lies in the use of antibodies, including the "B5 antibody", to the given antigen to agglutinate erythrocytes in a sample, as a diagnostic test for cancer in a subject from which the sample has been taken. This haemagglutination is described in the given publications.

### DESCRIPTION OF THE INVENTION

A novel antibody has a characteristic N-terminal amino-acid sequence in the λ light chain, i.e. Tyr-Glu-Leu-Ile-Gln-Pro-Pro-Ser-Ala-Ser-Val-Thr-Gln-Gly-Ala-Thr-Val-Ser-Leu-Tyr-Cys-Ser-Ile-. It has the characteristic already associated with the B5 antibody, i.e. causing haemagglutination of erythrocytes from patients with cancer to a substantially higher degree than of erythrocytes from non-cancer patients.

An antibody of the invention can be prepared in conventional manner from a rat hybridoma. A novel hybridoma producing such an antibody was deposited at the European Collection of Animal Cell Cultures, Porton Down, England, on 11 December 1987, and has the deposit number 87121101.

The reaction between the antibody and the antigen allows cancer to be detected indirectly, using a non-invasive technique and a simple test procedure. The correlation between B5 positive shift and active cancer is at least approx. 80%.

The novel antigen which, in vivo, is present as a minor component on the surface of human erythrocytes, and which becomes enhanced in cancer patients, has the following characteristics:

soluble in 1% deoxycholate;

in 1% deoxycholate, strongly binds the lectin Triticum vulgaris (Tv), while Phytolacca americana (Pa), Mycoplasma gallisepticum (Mg), Arachis hypogea (Ah), Helix pomatia (Hp) and Tetragonolobus paupurea - (Tp) do not bind; removal of deoxycholate results in aggregation of antigen: the aggregated form binds both Tv and Pa, but not Mg, Ah, Hp or Tp;

low affinity binding to concanvalain A and Lentil lectin (Lens culinaris);

trypsin-sensitive;

position after separation on Sephadex G200 column similar to that of IgG (mol. wt. 146,000) (run in 0.6% deoxycholate);

silver stain on analytical acrylamide gel; and

protein only just detectable (Petersen modification of Lowry method).

Trypsin-sensitivity and silver staining indicate that the novel antigen is a glycoprotein. The fact that Arachis hypogea binds to the Thomsen-Friedenreich (T) blood group antigen indicates that the novel antigen is not T-antigen. The lectins Helix pomatia and Tetragonolobus paupureas are respectively specific for A and H blood group antigens. Mycoplasma gallisepticum is specific for Glycophorin A. Phytolacca americana and Triticum vulgaris are given in the Sigma catalogue as lectins specific to the sugars (D-glcNAc)$_3$ and (D-glcNAc)$_2$NeuNAc respectively.

The novel antigen is a naturally-occurring blood group antigen. Controls (c.20%) may be naturally B5-positive (usually weak). Thus, a shift in B5-positivity is indicative of the presence of cancer in an individual; no reason other than cancer has been found for such a shift.

The novel antigen may be obtained by washing and then pooling erythrocytes from cancer patients. Erythrocyte ghosts are then prepared (by standard methods). The ghosts are dissolved in 1% deoxycholate and separated on a Sephadex G200 column; the antigen-containing fractions are eluted immediately after a large excluded fraction containing the majority of membrane proteins. Antigen is further purified by binding to immobilised lectin (Lens culinaris:recognises α-D-man: relatively low affinity for antigen, therefore possible to elute antigen off again by competition with α-methylmannoside). The purified antigen shows a molecular weight of c. 35,000 by SDS-PAGE.

In the haemagglutination test of the invention, a small volume, e.g. 1-5 ml, of blood containing anti-coagulant is taken. Washed erythrocytes (e.g.1% in PBS) are then mixed with an antibody of the invention, e.g. in a multi-well plate, and agglutination is observed in comparison with control experiments not using the antibody. For use in such a test, a diagnostic kit comprises the antibody (or other macromolecule, see below) against the antigen, and a plate or other support for the agglutination and control reactions.

For use in the test of the invention, any antibody to an antigen of the invention may be used, which recognises the same epitope as the B5 antibody, or a substantially equivalent epitope. The antibody may be a known macromolecule, e.g. an enzyme, other polypeptide, or lectin. A suitable lectin will bind to small carbohydrate groups, e.g. glucose dimers/trimers. It is of course important that the antibody which is used should be characterised by causing haemagglutination of erythrocytes from patients with cancer to a substantially higher degree than of erythrocytes from non-cancer patients. The B5 antibody appears to recognise a unique epitope on the B5 antigen; the epitope is certainly distinct from that seen by the lectin Triticum vulgaris. This can be demonstrated by immobilising the lectin, capture of monomeric B5 antigen, and chasing with $^{125}$I-B5 antibody.

The following Example 1 illustrates the preparation of the novel antigen from the novel antibody in more detail. Here and throughout, the following definitions apply:

P/NaCl = 5mM phosphate - 0.15 M sodium chloride, pH 8.0

SDS-PAGE = Sodium dodecylsulphate polyacrylamide gel electrophoresis

RBC = red blood cell

Protein A = Protein A from Staphylococcus aureus

DOC = deoxycholate

Example 1

Spin blood samples from B5 positive patients (keep separate to avoid cross-reactions) in bench centrifuge at 4°C, 3,000 rpm for 10 min. Remove supernatants and wash pellets with cold P/NaCl. Spin as above. Remove supernatant, and resuspend pellets in P/NaCl. Combine pellets for final wash and re-spin. Remove supernatant.

For lysis and preparation of erythrocyte ghosts: estimate packed rbc volume, and add x10 volume of cold P/NaCl. Leave on ice 10 min; transfer to centrifuge tubes and spin at 30,000 g at 4°C for 20 min. Discard supernatant. Collect ghosts, leaving behind small hard pellet below. Discard pellet. Replace ghosts, add more P/NaCl to fill tubes. Spin. Repeat procedure until ghosts are creamy/opaque.

Discard supernatant and resuspend ghosts in cold 10 mM Tris pH 8.2. Pool ghosts and spin out at 30,000g; ghosts may be stored at -20°C once they are opaque and clean. Control preparations utilised blood from B5 negative patients.

For membrane solubilisation: take 1 ml packed ghosts and add 2 ml 10 mM Tris-1% DOC (pH 8.2). Leave on ice for 15 min. Then remove any contaminant immunoglobulin which cross-reacts with protein A by incubation with Staphylococcus A (fixed; 10% suspension to give 1/50 of total volume). After 15 min incubation on ice, spin out bacteria and decant remaining solubilised membrane components.

For antigen isolation: covalently couple B5 antibody to Sepharose 4B-protein A using dimethylpimelimidate dihydrochloride according to the method of Schneider et al J. Biol. Chem. (1982) 257.

Wash affinity resin in 10 mM Tris-1% DOC before adding 100 $\mu$l resin to solubilised ghosts. Rotate at 4°C overnight. Spin out beads in microfuge at +4°C for 30 sec. Discard supernatant and add equivalent volume of ice-cold 10 mM Tris-1% DOC. Resuspend beads and microfuge. Repeat so beads have two washes in 10 mM Tris-1% DOC.

To dissociate antigen: incubate resin in 2 ml elution buffer (50 mM diethylamine hydrochloride, pH 11.4/0.5% DOC) at 37°C for 15 min. Spin out resin and collect eluted antigen in supernatant. Immediately neutralise eluate to pH 7.4 by addition of 0.5 mM $NaH_2PO_4$ (1/10).

By way of example of radioimmunoassay for the antigen, the following procedure was adopted:

Sensitise "high activated" titertec flexiplate with a 0.1 mg/ml Triticum vulgaris solution in PBSN⁻. 50 $\mu$l/well applied. Leave overnight at 4°C.

Wash plate x 2 with cold PBSN⁻ 2% BSA, 300 $\mu$l/well. Leave 2 hours.

Wash plate x2 with cold PBSN⁻. Apply samples (50 $\mu$l) diluted in PBSN⁻ 2% BSA. Duplicate wells done of each sample. Eluate titrations normally started at 1/8.

Wash plate x6 in cold PBSN⁻. Apply 50 $\mu$l of 1/1000 $^{125}$I-labelled B5 antibody in PBSN⁻ 2% BSA to wells. Leave 45 mins.

Wash plate x2 in cold PBSN⁻. Cut up plate and count individual wells in $\gamma$ counter. (1 min. counts for "quick" results, then 10 min. counts overnight).

The following Example 2 illustrates the haemagglutination test.

Example 2

A 5 ml sample of blood is collected into anti-coagulant (heparin). Appropriate conditions for sample storage were assessed by testing aliquots from samples kept at 4°C. Storage for up to 7 days in these conditions did not alter the B5-haemagglutination properties compared to the fresh sample.

For assay, 1 ml of whole blood was washed 3 times in 20 ml PBS, pH 7.4, at room temperature. A small volume of washed, packed erythrocytes was then diluted to 1% in PBS containing 4% foetal calf serum (FCS) and 25 $\mu$l B5 antibody in a "U" well haemagglutination plate. In controls, 1% erythrocytes were added to PBS containing 4% FCS, or to culture supernatant containing an irrelevant antibody. The plates were covered with film and left for at least 2 h at room temperature before being read.

Haemagglutination was scored positive or negative, using an inverted microscope to view the pellets directly under low power. Each sample was also scored by direct examination of cells gently resuspended and transferred onto a glass slide. In the latter method, samples of haemagglutinated clusters of less than about 20 cells were regarded as negative; the rest scored positive.

Trypsin treatment was with 0.125% trypsin in $Ca^{++}Mg^{++}$ free PBS at room temperature. Neuraminidase was used at 1 i.u. ml⁻¹ in 0.85% NaCl containing $10^{-3}$M $CaCl_2$ and incubated at 37°C for 2 h unless otherwise stated; this treatment causes extensive desialylation of the membrane surface.

In a preliminary clinical survey, it was found that the B5 haemagglutination test discriminates between erythrocytes from patients with malignant disease and erythrocytes from individuals with no known malignancy. Results showed that greater than about 80% of patients (total 386) with various types of cancer were B5 positive. These include each of 6 patients with tumours affecting the central nervous system, a tumour type rarely detected by other markers. Since the incidence of B5 positivity was 80% in tumour patients and about 20% in each of the control groups, it may be concluded that erythrocyte surface B5 antigen is markedly increased in individuals who develop malignant tumour. Preliminary results comparing women with benign and malignant breast disease showed a lower incidence of B5 positively in pre-operative samples from the benign group (4/8) in contrast to those with malignant disease (12/13).

Chemical Data

Over 50,000 haemagglutination tests have been done on patients and control sampled.
Tests have included:

```
*   A.   Breast cancer (600 patients)
    B.   Colo-rectal cancer (100 patients)
*   C.   Testicular cancer (150 patients)
    D.   Leukemia (100 children)
*   E.   Bladder cancer (200 patients)
*   F.   Lyniphomas (400 patients)
    *    The results are published or in preparation.
```

Control trials have been various, on over 3000 patients including 2000 radiotherapy patients having various types of tumour.

The following observations have been made:

A. Primary or recurrent cancer is the only known cause of significant positive shift in the B5 status of autologous erythrocytes.

B. Although B5 is a naturally-occurring red cell antigen, the large majority of individuals have low B5 level.

C. General practice patients with no known malignancy maintain a constant B5 status regardless of age or sex.

D. Successful treatment of tumour is associated with a reduction in B5 positivity. Recurrence is often associated with a positive B5 shift.

E. Early stage disease (such as stage one seminoma, Duke's B colo-rectal cancer, and low grade lymphoma) is often associated with an increased B5 status.

F. A lead time of 6 months has been found for some patients who develop recurrent tumour.

The novel antibody is apparently a universal cancer marker. Thus, it may be used to monitor disease status in cancer patients regardless of tumour type. A combination of the haemagglutination test with other tests such as using other markers, ESR or cytology, can markedly increase the specificity for disease. Enhanced specificity occurs in about 50% of patients.

**Claims**

1. An antigenic compound (as obtainable from humans where, in vivo, it is present as a minor component on the surface of erythrocytes, and which becomes enhanced in cancer patients), having the following characteristics:
soluble in 1% deoxycholate;
monoepitopic for wheat germ agglutinin (Triticum vulgaris);
binds Phytolacca americana and Triticum vulgaris;
does not bind Arachis hypogea, Helix pomatia, Mycoplasma gallisepticum or Tetragonolobus paupureas;
trypsin-sensitive;
molecular weight (monomer) of approx. 35,000 on sodium dodecylsulphate-polyacrylamide gel electrophoresis (SDS-PAGE);
silver stain on analytical acrylamide gel; and
protein only just detectable (Petersen modification of Lowry method).

2. A compound according to claim 1, which is antigenic to a monoclonal antibody as obtainable from hybridoma ECACC 87121101.

3. A monoclonal antibody to an antigen according to claim 1 or claim 2.

4. A monoclonal antibody according to claim 3, characterised by causing haemagglutination of erythrocytes from patients with cancer to a substantially higher degree than of erythrocytes from non-cancer patients.

5. A monoclonal antibody according to claim 4, immunoglobulin IgG subclass 2c, having $\lambda$ light chains.

6. A rat monoclonal antibody according to claim 5.

7. A monoclonal antibody according to any of claims 3 to 6, wherein the light chain N-terminal amino-acid sequence is Tyr-Glu-Leu-Ile-Gln-Pro-Pro-Ser-Ala-Ser-Val-Thr-Gln-Gly-Ala-Thr-Val-Ser-Leu-Tyr-Cys-Ser-Ile-.

8. A monoclonal antibody as obtainable from hybridoma ECACC 87121101.

9. Hybridoma ECACC 87121101.

10. A method of testing for cancer, which comprises agglutinating erythrocytes in a sample obtained from a subject by contacting the sample with a monoclonal antibody according to any of claims 3 to 8 or another antibody (or macromolecule acting as an antibody) to a compound according to claim 1 or claim 2.